# EUROPEAN PATENT APPLICATION

(11) **EP 4 059 434 A1**
(43) Date of publication of application: **21.09.2022**
(21) Application number: 22163025.4
(22) Date of filing: 18.03.2022
(51) Int. Cl.: A61B 6/00, G06N 3/02

(54) **DOSE ADJUSTMENT METHOD AND X-RAY SYSTEM**

(30) Priority: 19.03.2021 US 202117206866; 31.01.2022 JP 2022013103
(71) Applicant: Canon Medical Systems Corporation, Tochigi 324-0036 (JP)
(72) Inventor: BAUMGART, John, Tochigi (JP); MANAK, Joseph, Tochigi (JP)
(74) Representative: Marks & Clerk LLP

(57) **Abstract**

A dose adjustment method according to an embodiment includes irradiating a patient (P) with x-rays and obtaining an image (300) of the patient (P), creating a list of regions of interest (ROIs) related to the image (300) based on the image (300), creating a list of measurement fields of pixels corresponding to the created list of ROIs, providing to a user an interface (600) for selecting a measurement field from the created list of measurement fields, determining an x-ray dose of x-rays to be irradiated the patient (P), based on the selected measurement field, and irradiating the patient (P) with the determined x-ray dose.

## Description

### FIELD

Embodiments described herein relate generally to a dose adjustment method and an X-ray system.

Specifically, the present disclosure relates to the dose adjustment method and the X-ray system for displaying and selecting region of interest (ROI) for X-ray dose adjustment, and relates to adjusting x-ray dosage in x-ray imaging.

### BACKGROUND

In an x-ray image, there may be several objects that are of interest to a user at a given time, including anatomic markers or features, implanted devices, and interventional devices. The visibility of any of these is dependent on optimizing the x-ray dose to the patient for that object, which may compromise the visibility of other objects seen in the image. If it is not known what the primary object of interest is, it is possible that the x-ray dose is either too low or higher than is necessary to sufficiently resolve the particular object in an image.

Current practice relies on an overall or weighted regional measurement of an acquired image to determine the proper x-ray dose, but this disregards what a user is interested in seeing within an image with respect to the actual image content. In addition, current practice uses fixed regions of interest that may or may not have anything to do with patient anatomy or objects of interest therein. In general, in a section of a human body whose an x-ray image has been taken, there are several smaller sections mingled with other smaller sections, each of the various sections having intensity level different than the neighboring sections. A selected x-ray dose may not be the proper one for displaying the particular section a user wants to see in a high quality image.

### SUMMARY OF INVENTION

A dose adjustment method according to an aspect of the present disclosure includes irradiating a patient with x-rays and obtaining an image of the patient, creating a list of regions of interest (ROIs) related to the image based on the image, creating a list of measurement fields of pixels corresponding to the created list of ROIs, providing to a user an interface for selecting a measurement field from the created list of measurement fields, determining an x-ray dose of x-rays to be irradiated the patient, based on the selected measurement field, and irradiating the patient with the determined x-ray dose.

The creating a list of ROIs may be performed via a machine learning process

The creating a list of ROIs may be performed based on predetermined knowledge of the objects present in the image.

The list of ROIs may be presented concurrently with the image, along with indications as to the ROI currently being used to establish the measurement field for dose adjustment.

The measurement fields corresponding to particular ROIs may be indicated on the image, either temporarily or persistently.

A selection of a current ROI may be changed using an input interface including, a mouse, a joystick, a jog wheel, a touch panel, or voice control.

The machine learning process may be an object detection, localization, segmentation, or tracking process.

The creating a list of measurement fields of pixels may be performed based on the machine learning process.

The dose adjustment method may include determining a new corresponding measurement field, for determining the new x-ray dose, upon selection of a new ROI by the user.

ROIs may be selected by name via a menu.

The dimensions of the measurement fields of pixels corresponding to particular ROIs may be based on the type of objects present in the particular ROIs.

The obtaining the image may comprise irradiating the patient with the x-rays and obtaining an x-ray image of the patient.

The image may be fluoroscopic image.

The image may be two-dimensional image.

The x-ray dose may be adjusted based on statistical value using pixel values in the measurement field.

The x-ray dose may be adjusted based on comparison between statistical value using pixel values in the measurement field and threshold value.

The statistical value may be an average of pixel values.

The statistical value may be a weighted average of pixel values.

The predetermined knowledge may be information in which a device or the section of the patient is associated with image features in the image.

An x-ray system according to an aspect of the present disclosure includes an x-ray tube that irradiates a patient with x-rays and obtains an x-ray image of the patient, and processing circuitry configured to create a list of ROIs related to the x-ray image based on the x-ray image, create a list of measurement fields of pixels corresponding to the created list of ROIs, provide to a user an interface for selecting a measurement field from the created list of measurement fields, and determine an x-ray dose of x-rays to be irradiated the patient, based on the selected measurement field, wherein the x-ray tube irradiates the patient with the determined x-ray dose.

The processing circuitry may perform the creating a list of ROIs via a machine learning process.

The circuitry may perform the creating a list of ROIs based on predetermined knowledge of the objects present in the x-ray image.

The list of ROIs may be presented concurrently with the x-ray image, along with indications as to the ROI currently being used to establish the measurement field for dose adjustment.

The measurement fields corresponding to particular ROIs may be indicated on the x-ray image, either temporarily or persistently.

A selection of a current ROI may be changed using an input interface including, a mouse, a joystick, a jog wheel, a touch panel, or voice control.

The machine learning process may be an object detection, localization, segmentation, or tracking process.

The circuitry may perform the creating a list of measurement fields of based on the machine learning process.

The circuitry may determine a new corresponding measurement field, for determining the new x-ray adjustment, upon selection of a new ROI by the user.

ROIs may be selected by name via a menu.

The x-ray image may be fluoroscopic image.

The x-ray image may be two-dimensional image.

The x-ray dose may be adjusted based on statistical value using pixel values in the measurement field.

The x-ray dose may be adjusted based on comparison between statistical value using pixel values in the measurement field and threshold value.

The statistical value may be an average of pixel values.

The statistical value may be a weighted average of pixel values.

The predetermined knowledge may be information in which a device or the section of the patient is associated with image features in the x-ray image.

A dose adjustment method according to an aspect of the present disclosure includes accepting an operation of selecting a target object, imaging a section of a body of a patient and obtaining an image of the section, determining a region of interest (ROI) corresponding to the target object related to the obtained image, creating a measurement field of pixels based on the detected ROI, determining an x-ray dose for irradiating the section of the body of the patient with an x-ray, based on the created measurement field, and irradiating the section of the body of the patient with the determined x-ray dose.

A list of ROIs may be presented concurrently with the x-ray image, along with indications as to the ROI currently being used to establish the measurement field for dose adjustment determination.

The measurement fields corresponding to particular ROIs may be indicated on the x-ray image, either temporarily or persistently.

A selection of a current ROI may be changed using an input interface including, a mouse, a joystick, a jog wheel, a touch panel, or voice control.

The dose adjustment method may include determining a new corresponding measurement field, for determining the new x-ray dose, upon selection of a new ROI by the user.

ROIs may be selected by name via a menu.

The target object may be selected by a user and/or selected from medical records.

The adjusting determining an x-ray dose may be based on the selected target object associated with the created measurement field.

The detecting an ROI may be performed via a machine learning process.

The creating of a measurement field of pixels may be performed based on the machine learning process.

The dimensions of the measurement fields of pixels corresponding to particular ROIs may be based on the type of objects present in the particular ROIs.

The imaging a section of the body of the patient may comprise irradiating the section and obtaining an x-ray image of the section.

The image may be fluoroscopic image.

The image may be two-dimensional image.

The x-ray dose may be adjusted based on statistical value using pixel values in the measurement field.

The x-ray dose may be adjusted based on comparison between statistical value using pixel values in the measurement field and threshold value.

The statistical value may be an average of pixel values.

The statistical value may be a weighted average of pixel values.

The predetermined knowledge may be information in which a device or the section of the patient is associated with image features in the image.

This application presents a system and a method by which multiple regions or objects of interest can be indicated within an x-ray image, from which a user can select a primary region or object of interest. Identifying such regions or objects is done by the system with the assistance of, for example, a neural network. After the regions or objects are identified, the system provides the user the choice of a desired region or objects and sets the appropriate x-ray dose for the selected region or objects. The object of interest indicates the object itself, and the region of interest indicates the region corresponding to the object in the image.

### BRIEF DESCRIPTION OF THE DRAWINGS

The application will be better understood in light of the description which is given in a non-limiting manner, accompanied by the attached drawings in which:
Fig. 1 shows an example of the configuration of a photon-counting type X-ray CT apparatus.
Fig. 2 shows an example of the configuration of an X-ray diagnostic apparatus.
Figs. 3A, 3B show schematically a selection menu for selecting regions of interest.
Fig. 4 shows a block diagram indicating the steps in the process of assigning the right x-ray dose to a particular region of interest selected by a user.
Figs. 5A and 5B show an x-ray image with selected regions of interest.
Fig. 6 shows an interface flow-chart in one embodiment.
Fig. 7 shows an interface flow-chart in another embodiment.

### DETAILED DESCRIPTION

Reference throughout this specification to "one embodiment" or "an embodiment" means that a particular feature, structure, material, or characteristic described in connection with the embodiment is included in at least one embodiment of the application, but do not denote that they are present in every embodiment.

Thus, the appearances of the phrases "in one embodiment" or "in an embodiment" in various places throughout this specification are not necessarily referring to the same embodiment of the application. Furthermore, the particular features, structures, materials, or characteristics may be combined in any suitable manner in one or more embodiments.

The dose adjustment method and the x-ray system according to the present embodiments are applicable to various medical image diagnostic apparatuses that acquire medical image using x-rays. A specific example will be described below.

The operating environment of the present embodiments is described with respect to a computed tomography (CT) system. Moreover, the present embodiments will be described with respect to the detection and conversion of x-rays. However, one skilled in the art will further appreciate that the present embodiments are equally applicable for the detection and conversion of other high frequency electromagnetic energy, and also equally applicable with a wide variety of CT systems. That is, it is contemplated that the present embodiments may be utilized with energy integrating, photon counting (PC), and/or photon energy discriminating (ED) CT detector systems.

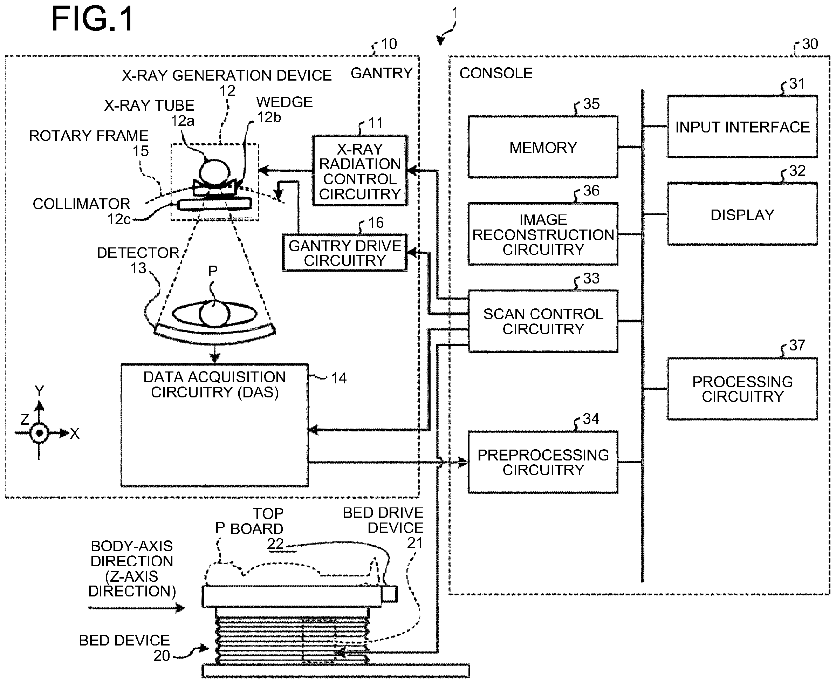

Fig. 1 is a diagram that illustrates an example of the configuration of a photon-counting type X-ray CT apparatus 1. As illustrated in Fig. 1, the photon-counting type X-ray CT apparatus 1 includes a gantry 10, a bed device 20, and a console 30.

The gantry 10 is a device that emits X-rays to a subject P (patient), detects the X-rays that are transmitted through the subject P, and outputs them to the console 30, and it includes X-ray radiation control circuitry 11, an X-ray generation device 12, a detector 13, data acquisition circuitry (DAS: Data Acquisition System) 14, a rotary frame 15, and gantry drive circuitry 16.

The rotary frame 15 is an annular frame that supports the X-ray generation device 12 and the detector 13 such that they are opposed to each other with the subject P interposed there between and that is rotated at high speed in a circular orbit around the subject P by the gantry drive circuitry 16.

The X-ray radiation control circuitry 11 is a device that serves as a high-voltage generation unit and supplies a high voltage to an X-ray tube 12a, and the X-ray tube 12a generates X-rays by using the high voltage that is supplied from the X-ray radiation control circuitry 11. Under the control of scan control circuitry 33, the X-ray radiation control circuitry 11 adjusts the tube voltage or the tube current that is supplied to the X-ray tube 12a, thereby adjusting the amount of X-rays that are emitted to the subject P.

Furthermore, the X-ray radiation control circuitry 11 switches a wedge 12b. Furthermore, the X-ray radiation control circuitry 11 adjusts the numerical aperture of a collimator 12c, thereby adjusting the radiation range (the fan angle or the cone angle) of X-rays. Moreover, there may be a case where multiple types of wedges are manually switched by an operator.

The X-ray generation device 12 is a device that generates X-rays and emits the generated X-rays to the subject P, and it includes the X-ray tube 12a, the wedge 12b, and the collimator 12c.

The X-ray tube 12a is a vacuum tube that emits X-ray beams to the subject P by using the high voltage that is supplied by the X-ray radiation control circuitry 11, and it emits X-ray beams to the subject P in accordance with the rotation of the rotary frame 15. The X-ray tube 12a generates X-ray beams that spread with the fan angle and the cone angle. For example, under the control of the X-ray radiation control circuitry 11, the X-ray tube 12a is capable of continuously emitting X-rays all around the subject P for a full reconstruction or continuously emitting X-rays for a half reconstruction within an emission range (180°+the fan angle) that enables a half reconstruction. Furthermore, under the control of the X-ray radiation control circuitry 11, the X-ray tube 12a is capable of intermittently emitting X rays (pulse X-rays) at a previously set position (tube position). Furthermore, the X-ray radiation control circuitry 11 is capable of changing the intensity of X-rays, emitted from the X-ray tube 12a. For example, the X-ray radiation control circuitry 11 increases the intensity of X-rays, emitted from the X-ray tube 12a, at a specific tube position, and it decreases the intensity of X-rays, emitted from the X-ray tube 12a, in the area other than the specific tube position.

The wedge 12b is an X-ray filter that adjusts the amount of X-rays with regard to the X-rays that are emitted from the X-ray tube 12a. Specifically, the wedge 12b is a filter that transmits and attenuates X-rays, emitted from the X-ray tube 12a, such that X-rays, emitted from the X-ray tube 12a to the subject P, has a predetermined distribution. For example, the wedge 12b is a filter that is obtained by processing aluminum so as to have a predetermined target angle or a predetermined thickness. Furthermore, the wedge is also called a wedge filter or a bow-tie filter.

The collimator 12c is a slit that narrows the irradiation range of X-rays, of which the amount of X-rays has been adjusted by the wedge 12b, under the control of the X-ray radiation control circuitry 11.

The gantry drive circuitry 16 drives and rotates the rotary frame 15 so that the X-ray generation device 12 and the detector 13 are rotated in a circular orbit around the subject P.

Each time an X-ray photon enters, the detector 13 outputs the signal with which the energy value of the X-ray photon may be measured. The X-ray photon is, for example, an X-ray photon that is emitted from the X-ray tube 12a and is transmitted through the subject P. The detector 13 includes multiple detection elements that output an electric signal (analog signal) of 1 pulse each time an X-ray photon enters. The photon-counting type X-ray CT apparatus 1 counts the number of electric signals (pulses) so as to count the number of X-ray photons that enter each of the detection elements. Furthermore, the photon-counting type X-ray CT apparatus 1 performs arithmetic processing on the signal so as to measure the energy value of the X-ray photon that causes output of the signal.

DAS 14 is configured to acquire projection data from X-ray detection data detected by the X-ray detector 13.

The couch device 20 is a device on which the subject P is placed and includes a couch driver 21 and a couchtop 22, as illustrated in FIG. 1. The couch driver 21 is configured to move the subject P into the rotating frame 15 by moving the couchtop 22 in a Z-axis direction. The couchtop 22 is a board on which the subject P is placed.

The console 30 is a device configured to receive operations performed by the operator on the X-ray CT apparatus 1 and also configured to reconstruct CT image data by using the projection data acquired by the gantry 10. As illustrated in FIG. 1, the console 30 includes an input interface 31, a display 32, scan control circuitry 33, preprocessing circuitry 34, memory 35, image reconstruction circuitry 36, and processing circuitry 37.

The input interface 31 includes a mouse, a keyboard, a trackball, a switch, a button, a joystick, and/or the like used by the operator of the X-ray CT apparatus 1 to input various types of instructions and various types of settings. The input interface 31 is configured to transfer information about the instructions and the settings received from the operator to the processing circuitry 37.

The display 32 is a monitor referenced by the operator and is configured to display a CT image generated from the CT image data for the operator and to display a Graphical User Interface (GUI) used for receiving the various types of instructions and the various types of settings from the operator via the input circuitry 31, under control of the processing circuitry 37.

Under the control of the processing circuitry 37, the scan control circuitry 33 is configured to control the projection data acquiring process performed by the gantry 10, by controlling operations of the X-ray radiation control circuitry 11, the gantry drive circuitry 16, DAS 14, and the couch driver 21.

The preprocessing circuitry 34 is configured to generate corrected projection data by performing a logarithmic converting process as well as correcting processes such as an offset correcting process, a sensitivity correcting process, a beam hardening correcting process, and the like, on the projection data generated by DAS 14.

The memory 35 is configured to store therein the projection data generated by the preprocessing circuitry 34.

The image reconstruction circuitry 36 is configured to reconstruct the CT image data by using the projection data stored in the memory 35.

The processing circuitry 37 is configured to exercise overall control of the X-ray CT apparatus 1 by controlling operations of the gantry 10, the couch device 20, and the console 30. Furthermore, the processing circuitry can execute various processes described below.

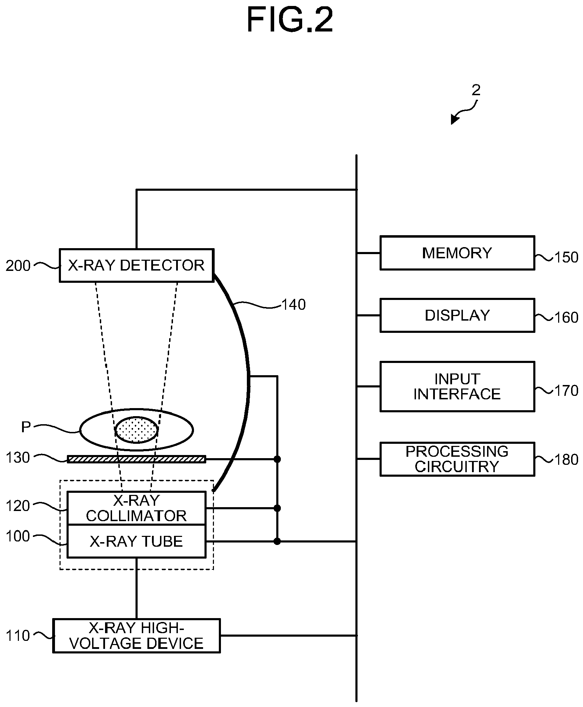

In addition, the operating environment of the present embodiments is described with respect to an X-ray diagnostic apparatus. Fig. 2 is a diagram that illustrates an example of the configuration of the X-ray diagnostic apparatus 2. As illustrated in FIG. 2, the X-ray diagnostic apparatus 2 includes an X-ray high-voltage device 110, an X-ray tube 100, an X-ray collimator 120, a table 130, a C-arm 140, an X-ray detector 200, a memory 150, a display 160, an input interface 170, and processing circuitry 180.

The X-ray high-voltage device 110 supplies a high voltage to the X-ray tube 100 under control of the processing circuitry 180.

The X-ray tube 100 generates an X-ray by irradiating thermions from a cathode to an anode by using a high voltage supplied from the X-ray high-voltage device 110.

The X-ray collimator 120 includes an X-ray beam aperture that limits an irradiation range of an X-ray generated by the X-ray tube 100, and a filter to adjust an X-ray emitted from the X-ray tube 100.

The table 130 is a bed on which the subject P is laid, and is arranged on a bed unit not illustrated.

The C-arm 140 holds the X-ray tube 100 and the X-ray collimator 120, and the X-ray detector 200, so as to oppose to each other sandwiching the subject P.

The X-ray detector 200 is an X-ray flat panel detector (FPD) having detecting devices that are arranged, for example, in a matrix. The X-ray detector 200 detects an X-ray that has been irradiated from the X-ray tube 100, and has passed through the subject P, and outputs a detection signal corresponding to a detected X-ray amount to the processing circuitry 180.

The memory 150 is implemented by, for example, a semiconductor memory device, such as a random access memory (RAM). The memory 150 stores a processing result by the processing circuitry 180. For example, the memory 150 accepts and stores various kinds of data, such as X-ray image data, collected by the processing circuitry 180. Furthermore, the memory 150 stores programs that correspond to respective functions read and executed by the processing circuitry 180.

The display 160 displays various kinds of information. For example, the display 160 displays a graphical user interface (GUI) to accept an instruction by an operator, or various kinds of X-ray images, under control of the processing circuitry 180. Moreover, the display 160 displays a processing result by the processing circuitry 180.

The input interface 170 accepts various kinds of input operations from an operator, and converts the accepted input operation into an electrical signal to output to the processing circuitry 180. For example, the input interface 170 is implemented by a mouse, a keyboard, a trackball, a switch, a button, a joystick, a touchpad with which an input operation is performed by touching an operating surface, a touch screen in which a display screen and a touchpad are integrated, a non-contact input circuit using an optical sensor, a voice input circuit, and the like. The input interface 170 may be constituted of a tablet terminal that is capable of wireless communication with an apparatus main unit, or the like. Furthermore, the input interface 170 is not limited to those having a physical operating part, such as a mouse and a keyboard. For example, a processing circuit of an electrical signal that receives an electrical signal corresponding to an input operation from an external input device separately provided from the apparatus, and outputs this electrical signal to the processing circuitry 180 is also included in examples of the input interface 170.

The processing circuitry 180 controls an overall operation of the X-ray diagnostic apparatus 2. The processing circuitry functions as various functions described below by reading and executing the programs stored in the memory 150. Hereinafter, various functions executed by the processing circuitry 180 will be described.

In one embodiment, a user is presented with information regarding image content that is used to influence the choice of an x-ray dose. Then, the user is given an interface (a feedback mechanism) for selecting a primary region of interest (ROI) from those objects detected to be present in the image content information. The selection of an object can then be used to create a new measurement field (i.e., a set of image pixels) that will be considered for setting the dose adjustment. That is, the processing circuitry 180 presents the interface (a list of the object of interest) for selecting a target and creates the measurement field according to the selected region of interest. The present embodiment is applicable to CT image and x-ray image. In addition, the present embodiment is applicable to various images including an acquisition image, a fluoroscopic image, a three-dimensional image, and a two-dimensional image.

In one embodiment, a list of objects of interest may be created from an object detection, localization, segmentation, or tracking process (which may include a neural network, or a "You Only Look Once" (YOLO) process, which is one of deep learning-based approaches in object detection).

In object detection, objects are identified in an image or video sequence. Objects can even be recognized when they are partially obstructed from view. This task is still a challenge for computer vision systems. Many approaches to the task can be implemented, including edge detection, appearance-based methods, and feature-based methods. The feature-based methods use information in which an object (a device or a section of the patient) is associated with image features in the image. In general, an object detection method falls into either a machine learning-based approach or a deep learning-based approach. Machine learning-based or deep learning-based approaches use trained models which can detect multiple kinds of objects.

In image segmentation, an image is partitioned into multiple regions according to some homogeneity criterion. In medical imaging, these segments often correspond to different tissue classes, organs, pathologies, or other biologically relevant structures. Different approaches have been developed for image segmentation, such as shape-based segmentation, image-based segmentation, interactive segmentation, and subjective surface segmentation.

This list of objects of interest may be presented concurrently with the x-ray image, along with indications as to the object currently being used to establish the measurement field for dose adjustment. The measurement field corresponding with said object is optionally indicated on the image, either temporarily or persistently. The image pixels comprising the measurement field are determined by the detection, localization, segmentation or tracking process. Typically (but not limited), the measurement field is set to include all of the ROI.

A measurement field typically differs from a region of interest. However, even if the measurement field and the region of interest comprise the same image pixels, each pixel of the measurement field may be weighted based on its importance. A pixel in the measurement field may be additionally weighted on how often or how recently it contributed to the measurement field.

An object in an image can be a region or "zone" of the image that has an overall intensity level different from other zones of the image. For example, in a coronary angiogram, one zone could be defined by low intensity levels, containing the spine and diaphragm, while another zone (e.g., the heart) would be defined by medium intensity values, and yet another (e.g., the lung) would be defined by high intensity values. The zone can be a contoured region determined by a machine-learning (ML) process, a bounding box or other stylized region of interest. The zone may be overlaid on the primary image or displayed in a separate graphical representation.

The dimensions of a measurement field may be derived from an ROI based on the existence (or not) of objects in a region of the ROI. The measurement fields may have a wide margin or a small margin depending on the object contained in the measurement field. The size of the margin may be defined either in absolute terms (e.g., millimeters) or relative terms (e.g., with respect to the contained object). Various examples are given below.

A measurement field containing a guide wire would typically have a rather wide margin, as guide wires are very small and tend to be moved around a lot. A large margin would allow such movement without affecting the correct determination of the measurement field.

A measurement field containing a stent would typically have a margin around the stent about the size of the stent itself. Stents typically don't move around within the patient once deployed, so the image information adjacent to the stent won't be unstable.

A measurement field containing a segment of spine would typically have a relatively small margin around the spine, as the spine is stable and is large enough on its own to give a reliably sized measurement field.

A measurement field containing an implanted device (e.g., pacemaker battery, artificial hip, dental work) would typically have little to no margin, as it is typically desired to exclude such a device from influencing dose calculations involving immediately adjacent measurement fields.

In cardiac images where there is periodic motion, a small measurement field around a specific object may move around the image from frame to frame. In this case, the measurement field may be an accumulation of measurement fields from several images to keep the dose stable.

The user is allowed to change the selection of the current primary object of interest (or region of interest) using an input device including, but not limited, to a mouse, joystick, jog wheel, touch panel, or voice control. Upon selection of a new primary ROI, the object tracking process is queried for the new measurement field. The new selection is indicated to the user, and the measurement field is then given to the x-ray dose adjustment process to determine the right x-ray dose for the selected new ROI.

Optionally, the user can manually select a ROI. This region can be selected tableside from a still frame or a live sequence displayed on a tablet (the user can use a gesture or ROI selection on the image presented).

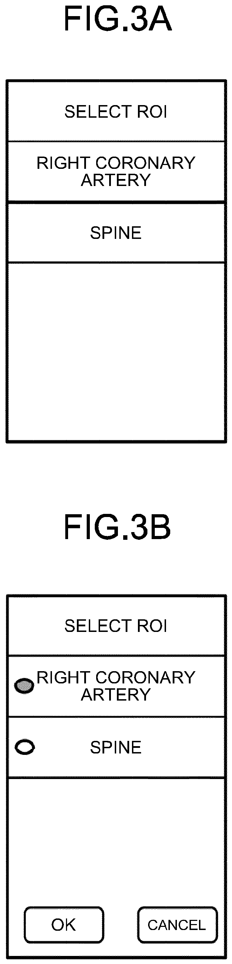

ROIs can be selected by name through a menu (including a drop down menu or a series of radio buttons), see Figs. 3A and 3B, or the ROIs can be presented on a touchpad and the user can select one.

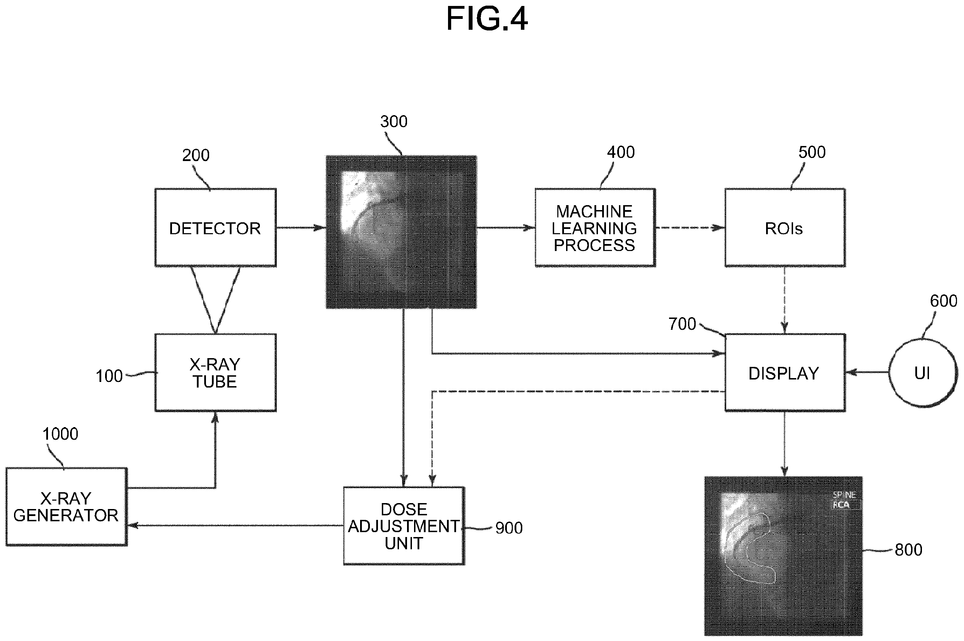

Fig. 4 shows a block diagram indicating the steps in one embodiment of a process of assigning the right x-ray dose to a particular ROI selected by the user. In Fig. 4, dashed lines refer to measurement fields, solid lines refer to image data and control information. First, the x-ray tube 100 irradiates an object with x-rays and via a detector200 an image 300 is displayed. Based on the image300, an ML process 400 is used to create a list of ROIs and respective measurement fields 500, i.e., outlines of a particular ROI in the image 300. Then, the user, via user interface (UI) 600, selects a measurement field of a particular ROI, and an ROI selection and display unit 700 displays an image 800, where the particular selected ROI is outlined for viewing by the user. For example, in the image 800, the region of interest is a right coronary artery (RCA) and the measurement field corresponding to RCA is indicated by a white curve. After the desired ROI has been selected, its information is provided to a dose adjustment unit 900 that sets the x-ray dose to correspond to the particular ROI and provides the information to the x-ray generator 1000 to irradiate the object with the adjusted x-ray. For example, the dose adjustment unit 900 adjusts the x-ray dose based on statistical value using pixel values in the measurement field. For example, the dose adjustment unit 900 adjusts the x-ray dose based on comparison between statistical value using pixel values in the measurement field and threshold value. In addition, the present embodiment is applicable to various statistical value including an average of pixel values and a weighted average of pixel values. Here, creating the outline of the ROI by the ML process 400, displaying user interface 600 and image 800 and controlling the x-ray dose by the dose adjustment unit 900 are performed by the processing circuitry. The x-ray generator 1000 corresponds to the x-ray high voltage device.

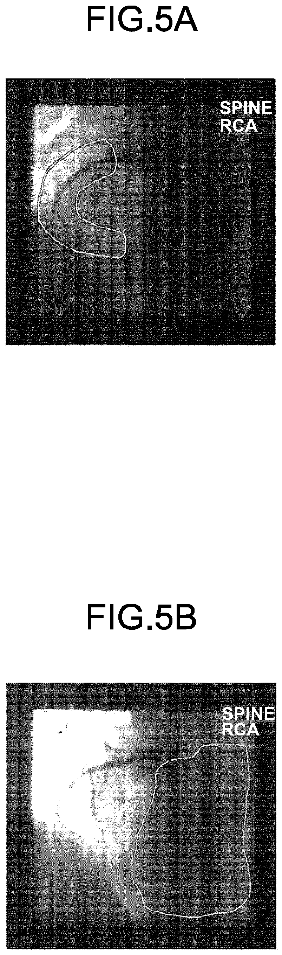

An example of a selection of a particular ROI in an x-ray image is shown in Figs. 5A and 5B. Figs. 5A and 5B show x-ray images from the same general region of the body. In Fig. 5A, a measurement field (white outline) corresponding to a particular ROI, i.e., a right coronary artery (RCA), is shown superimposed on the x-ray image. In Fig. 5B, a measurement field (white outline) corresponding to a particular ROI, i.e., the spine, is shown superimposed on the x-ray image. An indicator on the right upper corner of the screen informs the user about the identity of the selected ROI, i.e., RCA or spine. Then, the user selects one ROI, and the dose adjustment unit 900 provides the generator 1000 with the information, so that the generator 1000 selects the x-ray dose appropriate for the selected ROI, i.e., RCA or spine. This produces an x-ray image that has the best visibility for the particular ROI, i.e., RCA (Fig. 5A) or spine (Fig. 5B).

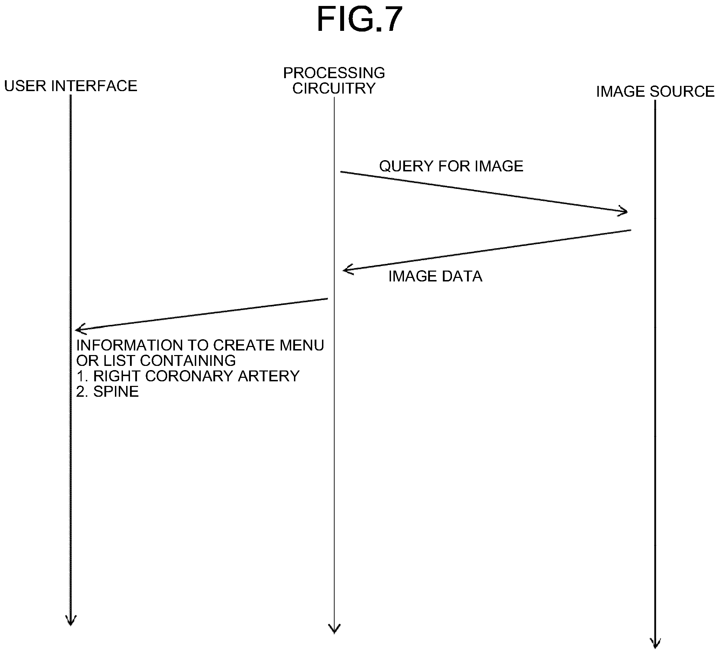

As seen in the interface flow-chart of Fig. 7, in this embodiment, the processing circuitry receives image data from the image source and generates information related to the image, i.e., list of measurement fields. It is noted that the image source may receive a query for an image from the processing circuitry.

In another embodiment, a list of objects of interest is created from a configured list of objects expected to be present in the image for the current acquisition. In particular, a list of target objects is selected. This list may be selected by a user from a populated database of medical images of the same or other users in medical records.

Image content can be inferred from, for example, the examination type (e.g., cardiac catheterization, electrophysiology, neuro, abdomen), the patient sex, or prior exam records (e.g., showing device implantation or vessel grafts). The examination type typically infers ROIs according to the following:
Cardiac:
Coronary arteries
   Right coronary
   Circumflex Artery
   Left Anterior Descending Artery
   Posterior Descending Artery
   Coronary stent
Catheter
   Guide Wire
   Vertebra
   Diaphragm
Electrophysiology:
   (same as cardiac)
   Ablation Catheter
   Pacemaker
Neuro:
   Carotid Artery
   Cerebral Artery
   Vertebral Artery
   Aneurysm coil
Abdomen:
   Catheter
   Guide Wire
   Aorta
   Ribs
   Diaphragm
   Spine
   Stent
   Renal Artery
   Mesenteric Artery
   Hepatic Artery

Then, based on the selected target object, a ROI is detected, via, for example, machine learning, and a measurement field of pixels based on the detected ROI is determined, via, for example, the machine learning process. Finally, based on the determined measurement field, the dose of an x-ray for irradiating the section of the body of the patient corresponding to the selected target object is adjusted.

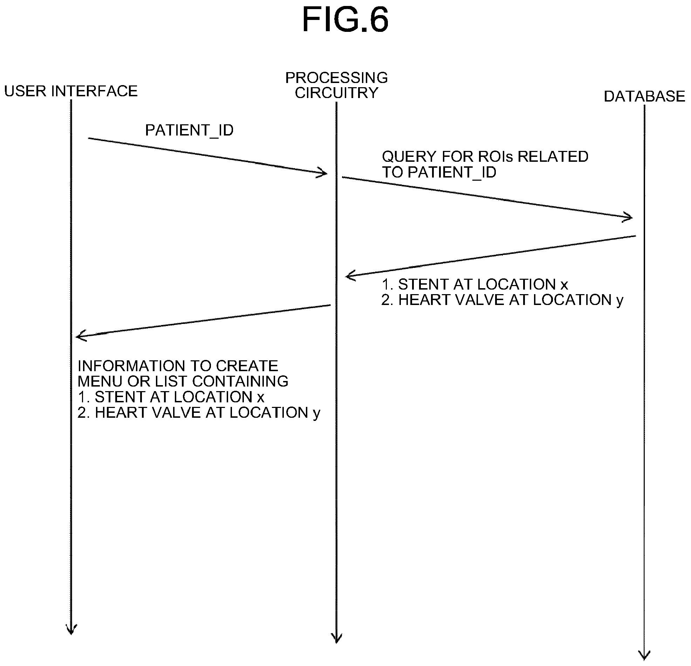

As seen in the interface flow-chart of Fig. 6, in this embodiment, the user interface provides the processing circuitry with user information (e.g., patient identification) and the processing circuitry queries the database for ROIs related to the patient ID. Information related to the patient (e.g., a stent located a position x or a heart valve located at a position y) is provided to the processing circuitry which generates a list of measurement fields outlining the stent at location x or the heart valve at location y.

The embodiments presented herein may be deployed in angiographic x-ray systems, although other x-ray applications are possible. Connections between components illustrated above may be the same as those existing in current such systems, but could be adapted to other hardware- or software-based connections.

This system advantageously determines regions of interest for x-ray dose adjustment based on image content rather than using histogram analysis of an arbitrary area. In this manner, the x-ray dose can be optimized for visualization of desired objects or regions within the field of view.

Various embodiments discussed herein provide a method for determining and selecting a particular ROI in an x-ray image for adjusting the x-ray dose for the particular ROI.

According to one embodiment, a section of a body of a patient is irradiated to obtain an x-ray image of the section and then a list of ROIs from the x-ray image is created and then a list of measurement fields of pixels corresponding to the created list of ROIs is created and selected, and an x-ray dose for irradiating the section of the body of the patient with an x-ray is adjusted, based on the selected measurement field.

According to one embodiment, the creating a list of ROIs is performed via a machine learning process.

According to another embodiment, the creating a list of ROIs is performed based on predetermined knowledge of the objects present in the x-ray image.

According to still another embodiment, the creating a list of ROIs is performed based on a list of target objects present in medical records and/or selected by a user.

According to one embodiment, the creating a list of measurement fields of pixels is performed based on the machine learning process.

Various embodiments discussed herein provide a system for determining and selecting a particular ROI in an x-ray image for adjusting the x-ray dose for the particular ROI.

According to one embodiment, the system comprises an x-ray tube that irradiates a section of a body of a patient and obtains an x-ray image of the section; and circuitry configured to: create a list of ROIs from the x-ray image; create a list of measurement fields of pixels corresponding to the created list of ROIs; and adjust an x-ray dose for irradiating the section of the body of the patient with an x-ray, based on the selected measurement field.

As described above, according to an embodiment, it is possible to set more appropriate an x-ray dose.

Those skilled in the art will also understand that there can be many variations made to the operations of the techniques explained above while still achieving the same objectives of the invention providing that such variations are within the scope of the appended claims. Such variations are intended to be covered by the scope of this disclosure. As such, the foregoing descriptions of embodiments of the invention are not intended to be limiting. Rather, any limitations to embodiments of the invention are defined by the appended claims.

## Claims

1. A dose adjustment method for adjusting imaging parameters, the dose adjustment method comprising:
irradiating a patient (P) with x-rays and obtaining an image (300) of the patient (P);
creating a list of regions of interest "ROIs" related to the image (300) based on the image;
creating a list of measurement fields of pixels corresponding to the created list of ROIs;
providing to a user an interface (600) for selecting a measurement field from the created list of measurement fields;
determining an x-ray dose of x-rays to be irradiated the patient (P), based on the selected measurement field; and
irradiating the patient (P) with the determined x-ray dose.

2. The dose adjustment method according to claim 1, wherein the creating a list of ROIs is performed via a machine learning process.

3. The dose adjustment method according to claim 1, wherein the creating a list of ROIs is performed based on predetermined knowledge of the objects present in the image (300).

4. The dose adjustment method according to any one of claims 1 to 3, wherein the list of ROIs is presented concurrently with the image (800), along with indications as to the ROI currently being used to establish the measurement field for dose adjustment.

5. The dose adjustment method according to any one of claims 1 to 4, wherein the measurement fields corresponding to particular ROIs are indicated on the image, either temporarily or persistently.

6. The dose adjustment method according to any one of claims 1 to 5, wherein a selection of a current ROI is changed using an input interface (600) including, a mouse, a joystick, a jog wheel, a touch panel, or voice control.

7. The dose adjustment method according to claim 2, wherein the machine learning process is an object detection, localization, segmentation, or tracking process.

8. The dose adjustment method according to claim 2, wherein the creating a list of measurement fields of pixels is performed based on the machine learning process.

9. The dose adjustment method according to claim 6, wherein upon selection of a new ROI by the user, the dose adjustment method further comprising determining a new corresponding measurement field, for determining the new x-ray dose.

10. The dose adjustment method according to claim 4, wherein ROIs are selected by name via a menu.

11. The dose adjustment method according to any one of claims 1 to 10, wherein the dimensions of the measurement fields of pixels corresponding to particular ROIs are based on the type of objects present in the particular ROIs.

12. The dose adjustment method according to any one of claims 1 to 11, wherein the obtaining the image (300) of the patient (P) comprises irradiating the patient with the x-rays and obtaining an x-ray image of the patient (P).

13. An x-ray system (1, 2) for determining and selecting a particular region of interest "ROI" in an x-ray image (300), for adjusting the x-ray dose for the particular ROI, the x-ray system (1, 2) comprising:
an x-ray tube (12a, 100) configured to irradiate a patient (P) with x-rays and obtains an x-ray image of the patient (P); and
processing circuitry (37, 180) configured to:
create a list of ROIs related to the x-ray image based on the x-ray image (300);
create a list of measurement fields of pixels corresponding to the created list of ROIs;
provide to a user an interface (600) for selecting a measurement field from the created list of measurement fields; and
determine an x-ray dose of x-rays to be irradiated the patient (P), based on the selected measurement field, wherein
the x-ray tube (12a, 100) is configured to irradiate the patient (P) with the determined x-ray dose.

14. The x-ray system (1, 2) according to claim 13, wherein the processing circuitry (37, 180) is configured to perform the creating a list of ROIs via a machine learning process.

15. The x-ray system (1, 2) according to claim 13, wherein the processing circuitry (37, 180) is configured to perform the creating a list of ROIs based on predetermined knowledge of the objects present in the x-ray image (300).
